# FASCICULE DE BREVET EUROPEEN

(11) **EP 1 516 610 B1**
(45) Date de publication et mention de la délivrance du brevet: **23.08.2006**
(21) Numéro de dépôt: 04292121.3
(22) Date de dépôt: 02.09.2004
(51) Int. Cl.: A61K 8/25, A61K 8/19, A61K 8/58, A61K 8/81, A61Q 19/00, A61Q 1/00, A61Q 19/08

(54) **Composition cosmétique contenant des particules colloidales de charge inorganique**
Kolloidale Teilchen als anorganischer Füllstoff enthaltendes kosmetisches Mittel
Cosmetic composition containing colloidal particles as inorganic filler

(30) Priorité: 17.09.2003 FR 0310915
(43) Date de publication de la demande: 23.03.2005
(73) Titulaire: L'ORÉAL, 75008 Paris (FR)
(72) Inventeur: Cassin, Guillaume, 91140 Villebon sur Yvette (FR); Roger, Véronique, 92220 Bagneux (FR)
(74) Mandataire: Renard, Emmanuelle

(56) Documents cités:
- EP-A- 0 312 270
- EP-A- 0 745 372
- DE-A- 10 142 216
- FR-A- 2 750 693
- FR-A- 2 783 419
- FR-A- 2 823 113
- US-A- 5 069 897
- US-A1- 2002 085 982
- DATABASE CAPLUS [Online] XP002276794 extrait de STN Database accession no. 2002:235865 & JP 2002 087929 A (KOSEI CO.) 27 mars 2002 (2002-03-27)

## Description

La présente invention se rapporte à une composition adaptée à une application topique sur la peau, renfermant une dispersion de particules colloïdales d'au moins une charge inorganique -en particulier de silice- dans un milieu physiologiquement acceptable contenant au moins une huile volatile et une huile non volatile, ladite composition étant exempte de cyclopentasiloxane.

Elle se rapporte aussi à l'utilisation cosmétique de la composition telle que définie précédemment pour estomper les imperfections cutanées, en particulier pour camoufler et/ou lisser les rides et ridules et/ou pour matifier la peau, ainsi qu'à un procédé cosmétique de soin ou de maquillage de la peau du visage et/ou du cou, en particulier d'une peau âgée ou d'une peau grasse, comprenant l'application sur ladite peau de la composition précitée.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge.

Il est connu de traiter ces signes du vieillissement en utilisant des compositions cosmétiques ou dermatologiques contenant des actifs capables de lutter contre le vieillissement, tels que les α-hydroxy-acides, les β-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés.

A cette fin, il a été proposé depuis quelques années des agents à effet tenseur qui lissent immédiatement après application les rides et ridules et contribuent à atténuer les marques de fatigue. Ces composés agissent en formant un film provoquant la rétraction du stratum corneum, la couche cornée superficielle de l'épiderme.

Parmi ces agents tenseurs, on trouve les particules colloïdales de charges inorganiques telles que la silice.

Si ces particules procurent effectivement un effet tenseur intéressant, elles présentent toutefois l'inconvénient de blanchir la peau lorsqu'elles sont formulées en présence d'huiles volatiles, ce qui nuit à leur utilisation dans des produits cosmétiques qui ne doivent pas laisser de trace inesthétique visible sur la peau après application.

Or, la Demanderesse a maintenant découvert qu'en formulant ces particules colloïdales en présence d'un mélange d'huiles volatiles et non volatiles, avantageusement dans un rapport en poids compris 1:1 et 3;1, il était possible d'obtenir des compositions cosmétiques à effet tenseur, permettant de lisser visiblement les rides et ridules sans blanchir la peau, et de surmonter ainsi les inconvénients de l'art antérieur.

Les documents US-3,819,825 , US-4,777,041 et US2002/0098220 divulguent, certes, l'utilisation d'une dispersion aqueuse de particules -colloïdales ou non- d'une charge minérale, en particulier la silice, pour lisser les rides par effet tenseur. Les compositions décrites dans ces documents sont toutefois exemptes d'huile.

D'autres documents décrivent des compositions anti-rides comprenant des particules - colloïdales ou non- de silice formulées en présence d'huile (FR-2 823 113 ; FR-2 659 551 ; WO 02/15873). Dans ces documents, les huiles peuvent être choisies parmi une longue liste d'huiles indifféremment volatiles et non volatiles. Il n'est cependant donné aucun exemple de formulation comprenant simultanément ces deux types d'huiles.

Or, la Demanderesse a montré que l'association d'huiles volatiles et non volatiles était indispensable à l'obtention d'une composition ne blanchissant pas à l'application tout en présentant un effet tenseur satisfaisant.

Les demandes FR-2 838 343 et FR-2 843 024 divulguent des compositions cosmétiques comprenant une dispersion colloïdale de particules inorganiques mélangée à des huiles volatiles et non volatiles. Ces compositions contiennent toutefois systématiquement du cyclopentasiloxane .

La présente invention a donc pour objet une composition adaptée à une application topique sur la peau, renfermant une dispersion de particules colloïdales, n'ayant pas de propriétés épaississantes, d'au moins une charge inorganique dans un milieu physiologiquement acceptable contenant au moins une huile volatile et une huile non volatile, ladite composition étant exempte de cyclopentasiloxane.

Par "particules colloïdales n'ayant pas de propriétés épaississantes" au sens de la présente invention, on entend une dispersion de particules ayant un diamètre moyen en nombre compris entre 0,1 et 100 nm, de préférence entre 3 et 30 nm, mieux, entre 10 et 15 nm. Ces particules conservent les diamètres précités dans la composition les contenant, sans s'aggréger, et n'ont donc pas de propriétés épaississantes en ce sens qu'à une concentration supérieure ou égale à 15% en poids dans tout solvant tel que l'eau, l'alcool ou l'huile, les particules colloïdales selon l'invention présentent une viscosité inférieure à 0,05 Pa.s pour un taux de cisaillement égal à 10⁻¹s, la viscosité étant mesurée à 25°C à l'aide d'un rhéomètre RheoStress RS150 de HAAKE en configuration cône-plan, le cône ayant un diamètre de 60 mm et un angle de 2°.

Cette dispersion de particules se distingue donc notamment des dispersions de particules de silice dite pyrogénée qui sont généralement obtenues selon un procédé en phase vapeur, par hydrolyse de tétrachlorure de silicium à très haute température et qui se présentent en réalité sous forme d'aggrégats, de sorte qu'elles sont improprement appelées "dispersions de silice colloïdale". Ainsi, lorsque la charge inorganique selon l'invention est constituée de silice, elle n'est pas pyrogénée.

La dispersion de particules colloïdales selon l'invention peut être préparée selon le procédé dit "sol-gel" bien connu de l'homme du métier, à partir de sels ou d'alcoxydes des métaux correspondants solubilisés dans un solvant, tels qu'un alcool. On procède ensuite à une réaction d'hydrolyse pour former un précipité amorphe. On disperse ensuite avec un acide ou une base selon le pH souhaité, ce qui conduit à la peptisation du précipité et à la cristallisation. On forme de cette façon un oxyde cristallin dans un solvant.

De telles suspensions colloïdales peuvent par exemple être préparées selon les procédés décrits dans *J*. *Colloïd Interface Sci*., 26, p. 62-69, 1968 pour SiO2, *Appli. Opt.* 26, 4688, 1987 pour TiO₂, *Inorg*. *Chem*., 3, 146, 1964 pour ZrO₂, *Appl*. *Opt*., 27, 3356, 1988 pour CaF₂ et MgF₂.

Ces suspensions sont préparées en utilisant des précurseurs ioniques choisis le plus souvent parmi les chlorures, les oxychlorures, les perchlorates, les nitrates, les oxynitrates ou encore les acétates ou des précurseurs moléculaires de préférence choisis parmi les alcoxydes, de formule molaire M(OR)ₙ (M représentant un métal, OR un radical alcoxy de 1 à 6 atomes de carbone et n représentant la valence du métal. Dans les méthodes décrites précédemment, le précurseur est hydrolysé ou fluoré puis polymérisé jusqu'à l'obtention d'un produit fini, insoluble dans le solvant choisi, nucléé et appelé suspension colloïdale. Dans le cas des alcoxydes, l'hydrolyse doit être rigoureusement contrôlée étant donné le caractère très hydrophile de ces dérivés organométalliques.

Un autre procédé de préparation de tels produits est décrit dans J. LIVAGE et autres, "SOL-GEL SYNTHESIS OF METAL OXYDE CLUSTERS AND COLLOIDS" (MAT. RES. SOC. SYNT. PROC., Volume 272, pages 3 à 14).

La charge inorganique utilisée dans la composition selon l'invention peut notamment être choisie parmi : la silice, l'oxyde de cérium, l'oxyde de zirconium, l'alumine, le carbonate de silicium, le sulfate de baryum, le sulfate de calcium, l'oxyde de zinc, le dioxyde de titane et le platine.

Comme exemples de silices colloïdales utilisables selon l'invention, on peut citer celles commercialisées par la société CATALYSTS & CHEMICALS sous les dénominations commerciales COSMO S-40® et COSMO S-50®.

Les particules colloïdales peuvent en variante être constituées d'un oxyde mixte, en particulier d'une charge composite silice-alumine.

Par "particules de charge composite silice-alumine" au sens de la présente invention, on entend des particules constituées d'oxyde de silicium et dont la surface a été modifiée chimiquement de façon à remplacer certains au moins des atomes de silicium par des atomes d'aluminium formant au plus une couche monomoléculaire d'aluminium. Leur portion de surface recouverte d'aluminium est généralement comprise entre 1 et 100%, de préférence entre 1 et 10%, mieux, entre 4 et 6%.

Ces particules ont généralement un potentiel zêta inférieur à -20 mV, et plus préférentiellement inférieur à -25 mV, à pH 7 et à 25°C, tel que mesuré à l'aide d'un appareil DELSA 440SX de COULTER Scientific Instrument.

Elles peuvent être préparées, notamment, comme décrit dans le brevet US-2,892,797, en mélangeant un sol de silice avec un aluminate de sodium. Elles sont par ailleurs disponibles dans le commerce auprès de la société GRACE sous les référence commerciales Ludox AM®, Ludox HSA® et Ludox TMA®.

Comme indiqué précédemment, la composition selon l'invention comprend un mélange d'huiles volatiles et non volatiles permettant d'éviter le blanchiment de la peau généralement observé lorsque les particules précitées sont appliquées sur la peau.

On entend par "huile" au sens de la présente invention une substance insoluble dans l'eau (sans modification de pH, à une teneur en matière active d'au moins 1% en poids à 25°C), qui est liquide à une température inférieure ou égale à 30°C, et qui n'est pas considérée comme un tensioactif dans l'un au moins des deux ouvrages suivants : Mc Cutcheon's : Emulsifiers and Detergents, International Edition, et International Cosmetic Ingredient Dictionary and Handbook (CTFA).

Par "huiles volatiles", on entend précisément des huiles ayant à une température de 20°C une pression de vapeur supérieure à 1 mbar. La pression de vapeur est définie comme la pression à laquelle un liquide et sa vapeur sont en équilibre à une température donnée. On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de "PF 5050®" et "PF 5060®" par la Société 3M et les dérivés de perfluoromorpholine, tels que la 4-trifluorométhyl perfluoromorpholine vendue sous la dénomination "PF 5052®" par la Société 3M.

Comme exemples d'huiles non volatiles, on peut citer : les huiles hydrocarbonées d'origine végétale ; les hydrocarbures linéaires ou ramifiés d'origine animale minérale ou synthétique ; les esters d'acides gras en C₈-C₃₀ et de mono-alcools en C₃-C₃₀ ; les esters d'hydroxyacides dont la chaîne acide et/ou alcool renferme de 8 à 29 atomes de carbone ; les esters de pentaérythrityle; les huiles fluorées ; les huiles de silicone ; et leurs mélanges.

Parmi les huiles hydrocarbonées d'origine végétale, on peut citer les triglycérides liquides d'acides gras comportant de 4 à 10 atomes de carbone comme les triglycérides des acides heptanoïque ou octanoïque ou encore, par exemple, les huiles végétales telles que les huiles de tournesol, de maïs, de soja, de courge, de pépins de raisin, de sésame, de noisette, d'abricot, de macadamia, d'ararachide, de tournesol, de ricin, d'avocat, l'huile de jojoba, l'huile de beurre de karité, ainsi que les triglycérides des acides caprylique/caprique comme ceux vendus par la société Stearineries Dubois ou ceux vendus sous les dénominations Miglyol 810, 812 et 818 par la société Dynamit Nobel.

Comme hydrocarbures d'origine animale, on peut citer le perhydrosqualène, et d'origine minérale ou synthétique, on peut citer les huiles de paraffine et leurs dérivés, la vaseline, les polydécènes, et le polyisobutène hydrogéné tel que l'huile de parléam.

D'autres huiles non volatiles utilisables dans la composition selon l'invention sont les esters et en particulier les esters d'acides gras en C₈-C₃₀ et de mono-alcools en C₃-C₃₀, tels que l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; ainsi que les esters d'hydroxyacides dont la chaîne acide et/ou alcool renferme de 8 à 29 atomes de carbone, tels que l'isostéaryl lactate, l'octylhydroxystéarate, l'hydroxystéarate d'octyldodécyle, le diisostéaryl-malate, le citrate de triisocétyle ; et les esters de pentaérythrityle comme le tétraisostéarate de pentaérythrityle.

En variante, les huiles non volatiles utilisées dans la composition selon l'invention peuvent être des alcools linéaires ou ramifiés ayant de 8 à 26 atomes de carbone, tels que l'octyldodécanol, le 2-butyloctanol, le 2-hexyldécanol, l'alcool oléique ou l'alcool linoléique.

Les huiles fluorées utilisables dans la composition selon l'invention peuvent être celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane.

Enfin, on peut citer comme huiles de silicone les composés à chaîne siliconée linéaire, notamment les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes.

La composition selon l'invention renferme de préférence de 0,5 à 80% en poids, de préférence de 1 à 55% en poids et, mieux, de 1 à 25% en poids au total d'huiles volatiles et non volatiles. Selon une forme d'exécution particulièrement préférée de l'invention, le rapport en poids des huiles volatiles aux huiles non volatiles est compris entre 1:1 et 3:1.

La composition selon l'invention est adaptée à une application topique sur la peau et comprend donc généralement un milieu physiologiquement acceptable, c'est-à-dire compatible avec la peau et/ou ses phanères. Ainsi, la composition selon l'invention a de préférence un pH inférieur à 8, mieux, inférieur ou égal à 7 et, encore mieux, compris entre 6 et 7.

La quantité de particules colloïdales présentes dans la composition peut varier dans une large mesure en fonction de l'effet recherché. A titre d'exemple, ces particules peuvent représenter de 0,01 à 15% en poids, et de préférence de 1 à 10% en poids, par rapport au poids total de la composition.

La composition selon l'invention peut se présenter sous toutes les formes galéniques classiquement utilisées pour une application topique et notamment sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E, E/H ou E/H/E.

Cette composition peut constituer un produit de soin du visage et/ou du cou. Elle peut en variante constituer un produit de maquillage tel qu'un fond de teint.

La composition selon l'invention peut en outre contenir divers adjuvants couramment utilisés dans le domaine cosmétique, tels que des émulsionnants, des charges, des conservateurs, des séquestrants, des colorants, des parfums, et des épaississants et gélifiants. Les quantités de ces différents adjuvants et leur nature seront choisis de manière à ne pas nuire aux propriétés de la composition selon l'invention, c'est-à-dire qu'ils n'affecteront pas négativement la capacité de tension de la peau de cette composition et ne laisseront pas subsister de dépôt blanchâtre sur celle-ci à l'application.

Selon une forme d'exécution avantageuse, la composition selon l'invention est exempte de glycérine. La Demanderesse a en effet constaté que l'effet tenseur des compositions selon l'invention était amélioré en l'absence de glycérine.

La Demanderesse a découvert que la composition selon l'invention était dotée de propriétés anti-rides par effet tenseur, ainsi que de propriétés optiques dites de "soft-focus" permettant d'envisager son utilisation pour matifier la peau, homogénéiser le teint et estomper les imperfections cutanées, tout en présentant un très bon toucher cosmétique.

L'invention a donc aussi pour objet un procédé cosmétique de soin ou de maquillage de la peau du visage et/ou du cou, en particulier d'une peau âgée ou d'une peau grasse, comprenant l'application sur ladite peau de la composition précitée.

Elle a aussi pour objet l'utilisation cosmétique de la composition définie précédemment pour lisser les rides et ridules et/ou retendre la peau du visage et/ou du cou.

Elle a aussi pour objet l'utilisation cosmétique de la composition telle que définie précédemment pour estomper ou camoufler les imperfections cutanées telles que les ridules, les pores, les irrégularités du micro-relief cutané et les taches pigmentaires.

Elle a encore pour objet l'utilisation cosmétique de la composition telle que définie précédemment pour matifier la peau.

Pour renforcer les effets anti-âge de la composition selon l'invention, celle-ci peut renfermer au moins un composé choisi parmi : les agents desquamants et/ou hydratants ; les agents dépigmentants ou propigmentants ; les agents anti-glycation ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-décontractants ; les agents anti-pollution et/ou anti-radicalaires; les agents amincissants ; les agents agissant sur la microcirculation ; les agents agissant sur le métabolisme énergétique des cellules ; et leurs mélanges.

Ainsi, la composition selon l'invention peut notamment contenir au moins un actif choisi parmi : les α-hydroxyacides ; l'acide salicylique et ses dérivés tels que l'acide n-octanoyl-5-salicylique ; l'HEPES ; l'acide L-2-oxothiazolidine-4-carboxylique ; l'O-octanoyl-6-D-maltose ; le sel disodique d'acide méthyl glycine diacétique ; les céramides ; les stéroïdes tels que la diosgénine et les dérivés de la DHEA ; la niacinamide ; l'acide ascorbique et ses dérivés ; les extraits de myrtille ; les rétinoïdes et en particulier le rétinol et ses esters ; les polypeptides et leurs dérivés acylés ; les phytohormones ; les extraits de levure Saccharomyces cerevisiae ; les extraits d'algues ; les extraits de Vitreoscilla filiformis ; les extraits de soja, de lupin, de maïs et/ou de pois ; l'alvérine et ses sels, en particulier le citrate d'alvérine ; le resvératrol ; les caroténoïdes et en particulier le lycopène ; le co-enzyme Q10 ou ubiquinone ; les xanthines et en particulier la caféine et les extraits naturels en contenant ; les extraits de petit houx et de marron d'Inde ; et leurs mélanges, sans que cette liste soit limitative.

La composition selon l'invention peut en outre contenir au moins un filtre UVA et/ou UVB. Les filtres solaires peuvent être choisis parmi les filtres organiques, les filtres inorganiques et leurs mélanges.

Dans le cas où elle est utilisée dans le traitement des peaux grasses, la composition selon l'invention peut comprendre au moins un actif choisi parmi : les agents desquamants ; les agents anti-séborrhéiques, en particulier les inhibiteurs de 5-réductase ; les agents apaisants ; les agents anti-bactériens ; et leurs mélanges.

Ces actifs peuvent être choisis notamment parmi : les rétinoïdes et en particulier le rétinol ; les sels de zinc tels que le gluconate de zinc ; un extrait de Laminaria saccharina ; le triclosan ; le phénoxyéthanol ; l'octoxyglycérine ; l'octanoylglycine ; le caprylyl glycol ; l'acide azélaïque ; les α-hydroxyacides tels que les acides lactique ou glycolique : les β-hydroxyacides, en particulier l'acide salicylique et ses dérivés tels que l'acide n-octanoyl-5-salicylique ; l'acide ursolique ; le panthénol ; et l'octopirox.

La composition selon l'invention peut également comprendre, outre les particules colloïdales de charge inorganique, diverses charges absorbant le sébum et/ou à effet optique.

Au sens de l'invention, « charge à effet optique » désigne une charge présentant un indice de réfraction inférieur ou égal à 2,2, notamment inférieur ou égal à 2, et en particulier inférieur ou égal à 1,8, de préférence allant de 1,3 à 1,6.

Plus particulièrement, ces charges à effet optique peuvent par exemple être choisie parmi :
- les poudres de polyamide (Nylon®), comme par exemple les particules de Nylon 12 du type Orgasol d'Atofina de taille moyenne 10 microns et d'indice de réfraction 1,54 ;
- les poudres de silice, comme par exemple ies Silica beads SB150 de Miyoshi de taille moyenne 5 microns et d'indice de réfraction 1,45 ;
- les poudres de polytétrafluoroéthylène, comme les PTFE ceridust 9205F de Clariant de taille moyenne 8 microns et d'indice de réfraction 1,36 ;
- les poudres de résine de silicone comme les Silicon resin Tospearl 145A de GE Silicone de taille moyenne 4,5 microns et d'indice de réfraction 1,41 ;
- les poudres de copolymères acryliques, notamment de poly(méth)acrylate de méthyle, comme les particules PMMA Jurymer MBI de Nihon Junyoki de taille moyenne 8 microns et d'indice de réfraction 1,49 ;
- les poudres de cire comme les particules Paraffin wax microease 114S de micropowders de taille moyenne 7 microns et d'indice de réfraction 1,54 ;
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48) ;
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations « KSP-100 », « KSP-101 », « KSP-102 », « KSP-103 », « KSP-104 », « KSP-105 » par la société SHIN ETSU ;
- les poudres composites de talc/dioxyde de titane/alumine/silice comme celles vendues sous la dénomination Coverleaf AR-80 par la société Catalyst & chemicals ; et
- leurs mélanges.

On entend par « composé absorbant le sébum », un composé apte à absorber et/ou adsorber le sébum, ayant une prise de sébum généralement supérieure ou égale à 1 ml/g, notamment supérieure ou égale à 2 ml/g, et en particulier supérieure ou égale à 3 ml/g.

Les particules de composé absorbant et/ou adsorbant le sébum peuvent être d'origine minérale ou organique.

Plus précisément, ce composé peut être choisi parmi la silice pyrogénée, les poudres de polyamide (nylon®), les poudres de polymères acryliques, notamment de polyméthacrylate de méthyle, de polyméthacrylate de méthyle/diméthacrylate d'éthylène glycol, de polyméthacrylate d'allyle/diméthacrylate d'éthylène glycol et de copolymère diméthacrylate d'éthylène glycol/méthacrylate de lauryle, et leurs mélanges.

La composition selon l'invention peut en outre renfermer diverses charges additionnelles d'origine minérale ou organique. Elles peuvent être de toute forme, notamment plaquettaires, sphériques ou oblongues, quelle que soit leur forme cristallographique (par exemple feuillet, cubique, hexagonale, orthorhombique, etc...).
Parmi les charges additionnelles utilisables dans la composition selon l'invention, on peut notamment citer le talc, le mica, le kaolin, de poly-β-alanine et de polyéthylène, la lauroyl-lysine, l'amidon, le nitrure de bore, le carbonate de calcium précipité, le carbonate et l'hydro-carbonate de magnésium, le sulfate de baryum, l'hydroxyapatite, les microcapsules de verre ou de céramique et les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium.

La composition selon l'invention peut avantageusement contenir en outre au moins une matière colorante pulvérulente pouvant être choisie parmi les pigments et les nacres habituellement utilisés dans les compositions cosmétiques et/ou dermatologiques.

Les pigments peuvent être blancs ou colorés, minéraux et/ou organiques, enrobés ou non. On peut notamment citer parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut notamment citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

L'invention sera maintenant illustrée par les exemples non limitatifs suivants. Dans ces exemples, les quantités sont indiquées en pourcentage pondéral et les composés sont généralement identifiés selon la nomenclature CTFA.

### EXEMPLES

### Exemple 1 : Composition cosmétique sous forme d'émulsion H/E

| *Phase A* | |
|---|---|
| Dimyristyl Tartrate (et) Cetearyl Alcohol (et) C₁₂₋₁₅ Pareth-7 (et) PPG-25-Laureth-25 | 1.50 g |
| Glyceryl Stearate (et) PEG-100 stearate | 2.00 g |
| Alcool Stearylique | 1.00 g |
| Cyclohexasiloxane | 7.50 g |
| Polyisobutène hydrogéné | 2.50 g |
| | |

| *Phase B* | |
|---|---|
| Conservateurs | 1.00 g |
| Ammonium polyacryloyldimethyl taurate | 0.40 g |
| Eau | 66.75 g |
| Gomme de xanthane | 0.20 g |
| Pentasodium ethylene diamine tetramethylene phosphonate | 0.05 g |
| | |

| *Phase C* | |
|---|---|
| Dispersion colloïdale de particules de silice | 17.10 g |
| (COSMO S-40 de Catalysts & Chemicals) | |

### Mode opératoire :

La composition ci-dessus a été préparée de la manière suivante : on a chauffé la phase B à environ 75°C et on y a incorporé l'ammonium polyacryloyldimethyl taurate, puis on a agité jusqu'à obtention d'un gel homogène. Séparément, on a chauffé la phase A à environ 75°C. On a ensuite réalisé l'émuision en incorporant la phase A dans la phase B, puis on a incorporé à 40-45°C la phase C en maintenant l'agitation jusqu'à refroidissement complet.

### Exemple 2 (comparatif) : Composition cosmétique sous forme d'émulsion H/E

| *Phase A* | |
|---|---|
| Dimyristyl Tartrate (et) Cetearyl Alcohol (et) | |
| C₁₂₋₁₅ Pareth-7 (et) PPG-25-Laureth-25 | 1.50 g |
| Glyceryl Stearate (et) PEG-100 stearate | 2.00 g |
| Alcool Stearylique | 1.00 g |
| Cyclohexasiloxane | 10.00 g |
| | |

| *Phase B* | |
|---|---|
| Conservateurs | 1.00 g |
| Ammonium polyacryloyldimethyl taurate | 0.40 g |
| Eau | 66.75 g |
| Gomme de xanthane | 0.20 g |
| Pentasodium ethylene diamine tetramethylene phosphonate | 0.05 g |
| | |

| *Phase C* | |
|---|---|
| Dispersion colloïdale de particules de silice | 17.10 g |
| (COSMO S-40 de Catalysts & Chemicals) | |

La composition ci-dessus a été préparée comme décrit à l'Exemple 1.

### Exemple 3 : Evaluation de l'effet blanchissant

Les compositions des Exemples 1 et 2 ont été testées sur une zone du dos de la main à raison d'environ 5 mg/cm². Trente minutes après l'application, une photographie des zones traitées a été prise, qui est présentée à la Figure annexée.

Comme le montre cette figure, la composition de l'Exemple 1 selon l'invention ne laisse pas subsister de dépôt blanc sur la peau après l'application (main droite), contrairement à la composition de l'Exemple comparatif 2 (main gauche).

### Exemple 4 : Evaluation des propriétés optiques in vivo

Les effets anti-rides, "soft-focus" et matifiant de la composition de l'Exemple 1 ont été évalués sur un panel de 12 femmes. Il a été constaté que cette composition diminuait la visibilité du micro-relief de la peau en affinant le grain de peau et matifiait instantanément les reflets et ce, de façon significative jusqu'à 4 heures après l'application. Simultanément, le teint paraissait plus unifié et les rides et ridules estompées. Aucun blanchiment de la peau n'a été mis en évidence.

### Exemple 5 : Evaluation de l'effet tenseur in vivo

La composition de l'Exemple 1 a été appliquée sur un panel de femmes âgées de 40 à 60 ans, présentant des rides et ridules au niveau du contour de l'oeil (étude par photographies bilatérales). Il a été observé un effet significatif de lissage des ridules se trouvant sous l'oeil et des rides de la patte d'oie après application de la composition, sans qu'aucun problème de blanchiment inesthétique n'ait été constaté.

## Revendications

1. Composition adaptée à une application topique sur la peau, renfermant une dispersion de particules colloïdales, n'ayant pas de propriétés épaississantes, d'au moins une charge inorganique dans un milieu physiologiquement acceptable contenant au moins une huile volatile et une huile non volatile, ladite composition étant exempte de cyclopentasiloxane.

2. Composition selon la revendication 1, **caractérisée en ce que** ladite charge inorganique est choisie parmi : la silice, l'oxyde de cérium, l'oxyde de zirconium, l'alumine, le carbonate de silicium, le sulfate de baryum, le sulfate de calcium, l'oxyde de zinc, le dioxyde de titane et le platine.

3. Composition selon la revendication 1, **caractérisée en ce que** ladite charge inorganique est la silice non pyrogénée.

4. Composition selon la revendication 1 ou 2, **caractérisée en ce que** ladite charge inorganique est un oxyde mixte.

5. Composition selon la revendication 4, **caractérisée en ce que** ladite charge inorganique est une charge composite silice-alumine.

6. Composition selon la revendication 5, **caractérisée en ce que** la surface desdites particules qui est recouverte d'aluminium est comprise entre 4 et 6%.

7. Composition selon la revendication 5 ou 6, **caractérisée en ce que** lesdites particules ont un potentiel zêta inférieur à -25 mV, à pH 7 et à 25°C.

8. Composition selon l'une quelconque des revendications 1 à 7, **caractérisée en ce que** lesdites particules ont un diamètre moyen en nombre compris entre 0,1 et 100 nm.

9. Composition selon la revendication 8, **caractérisée en ce que** lesdites particules ont un diamètre moyen en nombre compris entre 3 et 30 nm.

10. Composition selon l'une quelconque des revendications 1 à 9, **caractérisée en ce qu'**elle a un pH inférieur ou égal à 7.

11. Composition selon la revendication 10, **caractérisée en ce qu'**elle a un pH compris entre 6 et 7.

12. Composition selon l'une quelconque des revendications 1 à 11, **caractérisée en ce qu'**elle renferme de 1 à 10% en poids de particules colloïdales, par rapport au poids de la composition.

13. Composition selon l'une quelconque des revendications 1 à 12, **caractérisée en ce qu'**elle est exempte de glycérine.

14. Composition selon l'une quelconque des revendications 1 à 13, **caractérisée en ce que** ladite huile non volatile est choisie parmi : les huiles hydrocarbonées d'origine végétale ; les hydrocarbures d'origine animale, minérale ou synthétique ; les esters d'acides gras en C₈-C₃₀ et de mono-alcools en C₃-C₃₀ ; les esters d'hydroxyacides dont la chaîne acide et/ou alcool renferme de 8 à 29 atomes de carbone ; les esters de pentaérythrityle ; les alcools linéaires ou ramifiés ayant de 8 à 26 atomes de carbone ; les huiles fluorées , les huiles de silicone ; et leurs mélanges.

15. Composition selon l'une quelconque des revendications 1 à 14, **caractérisée en ce que** ladite huile volatile est choisie parmi : les silicones cycliques, les silicones linéaires renfermant de 2 à 6 atomes de silicium, les hydrocarbures ramifiés, les perfluoroalcanes et les dérivés de perfluoromorpholine.

16. Composition selon l'une quelconque des revendications 1 à 15, **caractérisée en ce qu'**elle renferme de 1 à 25% en poids au total d'huiles volatiles et non volatiles.

17. Composition selon l'une quelconque des revendications 1 à 16, **caractérisée en ce que** le rapport en poids des huiles volatiles aux huiles non volatiles est compris entre 1:1 et 3:1.

18. Composition selon l'une quelconque des revendications 1 à 17, **caractérisée en ce qu'**elle se trouve sous la forme d'une émulsion H/E, E/H ou E/H/E.

19. Procédé cosmétique de soin ou de maquillage de la peau du visage et/ou du cou, en particulier d'une peau âgée ou d'une peau grasse, comprenant l'application sur ladite peau de la composition selon l'une quelconque des revendications 1 à 18.

20. Utilisation cosmétique de la composition selon l'une quelconque des revendications 1 à 18 pour estomper ou camoufler les imperfections cutanées telles que les ridules, les pores, les irrégularités du micro-relief cutané et les taches pigmentaires.

21. Utilisation cosmétique de la composition selon l'une quelconque des revendications 1 à 18 pour matifier la peau.

22. Utilisation cosmétique de la composition selon l'une quelconque des revendications 1 à 18 pour lisser les rides et ridules et/ou retendre la peau du visage et/ou du cou.

## Claims

1. Composition suitable for topical application to the skin, containing a dispersion of colloidal particles, which do not have thickening properties, of at least one inorganic filler in a physiologically acceptable medium containing at least one volatile oil and one nonvolatile oil, the said composition being free of cyclopentasiloxane.

2. Composition according to Claim 1, **characterized in that** the said inorganic filler is chosen from: silica, cerium oxide, zirconium oxide, alumina, silicon carbonate, barium sulphate, calcium sulphate, zinc oxide, titanium dioxide and platinum.

3. Composition according to Claim 1, **characterized in that** the said inorganic filler is non-fumed silica.

4. Composition according to Claim 1 or 2, **characterized in that** the said inorganic filler is a mixed oxide.

5. Composition according to Claim 4, **characterized in that** the said inorganic filler is a silica-alumina composite filler.

6. Composition according to Claim 5, **characterized in that** the surface of the said particles which is covered with aluminium is between 4 and 6%.

7. Composition according to Claim 5 or 6, **characterized in that** the said particles have a zeta potential of less than -25 mV, at pH 7 and at 25°C.

8. Composition according to any one of Claims 1 to 7, **characterized in that** the said particles have a mean diameter in numerical terms of between 0.1 and 100 nm.

9. Composition according to Claim 8, **characterized in that** the said particles have a mean diameter in numerical terms of between 3 and 30 nm.

10. Composition according to any one of Claims 1 to 9, **characterized in that** it has a pH of less than or equal to 7.

11. Composition according to Claim 10, **characterized in that** it has a pH of between 6 and 7.

12. Composition according to any one of Claims 1 to 11, **characterized in that** it contains from 1 to 10% by weight of colloidal particles, relative to the weight of the composition.

13. Composition according to any one of Claims 1 to 12, **characterized in that** it is free of glycerine.

14. Composition according to any one of Claims 1 to 13, **characterized in that** the said nonvolatile oil is chosen from: hydrocarbon oils of plant origin; hydrocarbons of animal, inorganic or synthetic origin; esters of C₈-C₃₀ fatty acids and C₃-C₃₀ monoalcohols; esters of hydroxy acids in which the acid and/or alcohol chain contains from 8 to 29 carbon atoms; pentaerythrityl esters; linear or branched alcohols having from 8 to 26 carbon atoms; fluorinated oils; silicone oils; and mixtures thereof.

15. Composition according to any one of Claims 1 to 14, **characterized in that** the said volatile oil is chosen from: cyclic silicones, linear silicones containing from 2 to 6 silicon atoms, branched hydrocarbons, perfluoroalkanes and perfluoromorpholine derivatives.

16. Composition according to any one of Claims 1 to 15, **characterized in that** it contains from 1 to 25% by weight in total of volatile or nonvolatile oils.

17. Composition according to any one of Claims 1 to 16, **characterized in that** the weight ratio of volatile oils to nonvolatile oils is between 1:1 and 3:1.

18. Composition according to any one of Claims 1 to 17, **characterized in that** it is in the form of an O/W, W/O or W/O/W emulsion.

19. Cosmetic process for care or makeup of the face and/or the neck, in particular of an aged skin or of a greasy skin, comprising the application to the said skin of the composition according to any one of Claims 1 to 18.

20. Cosmetic use of the composition according to any one of Claims 1 to 18, for softening or concealing skin imperfections such as fine lines, pores, irregularities of the skin microrelief and pigmented spots.

21. Cosmetic use of the composition according to any one of Claims 1 to 18, for mattifying the skin.

22. Cosmetic use of the composition according to any one of Claims 1 to 18, for smoothing wrinkles and fine lines and/or retightening the skin of the face and/or of the neck.

## Patentansprüche

1. Zusammensetzung, die für eine topische Anwendung auf die Haut geeignet ist und eine Dispersion von keine verdickenden Eigenschaften aufweisenden kolloidalen Partikeln mindestens eines anorganischen Füllstoffs in einem physiologisch akzeptablen Medium enthält, welches zumindest ein flüchtiges Öl und ein nicht flüchtiges Öl enthält, wobei in der Zusammensetzung kein Cyclopentasiloxan enthalten ist.

2. Zusammensetzung nach Anspruch 1, **dadurch gekennzeichnet, dass** der anorganische Füllstoff ausgewählt ist unter: Kieselsäure, Ceroxid, Zirconiumoxid, Aluminiumoxid, Siliciumcarbonat, Bariumsulfat, Calciumsulfat, Zinkoxid, Titanoxid und Platin.

3. Zusammensetzung nach Anspruch 1, **dadurch gekennzeichnet, dass** der anorganische Füllstoff eine nicht pyrogene Kieselsäure ist.

4. Zusammensetzung nach Anspruch 1 oder 2, **dadurch gekennzeichnet, dass** der anorganische Füllstoff ein gemischtes Oxid ist.

5. Zusammensetzung nach Anspruch 4, **dadurch gekennzeichnet, dass** der anorganische Füllstoff ein zusammengesetzter Kieselsäure-Aluminiumoxid-Füllstoff ist.

6. Zusammensetzung nach Anspruch 5, **dadurch gekennzeichnet, dass** die Oberfläche der Partikel, die mit Aluminium bedeckt ist, im Bereich von 4 bis 6 % liegt.

7. Zusammensetzung nach Anspruch 5 oder 6, **dadurch gekennzeichnet, dass** die Partikel bei pH 7 und 25 °C ein Zeta-Potential unter -25 mV aufweisen.

8. Zusammensetzung nach einem der Ansprüche 1 bis 7, **dadurch gekennzeichnet, dass** die Partikel einen zahlenmittleren Durchmesser von 0,1 bis 100 nm aufweisen.

9. Zusammensetzung nach Anspruch 8, **dadurch gekennzeichnet, dass** die Partikel einen zahlenmittleren Durchmesser von 3 bis 30 nm aufweisen.

10. Zusammensetzung nach einem der Ansprüche 1 bis 9, **dadurch gekennzeichnet, dass** sie einen pH-Wert von 7 oder darunter aufweist.

11. Zusammensetzung nach Anspruch 10, **dadurch gekennzeichnet, dass** sie einen pH-Wert von 6 bis 7 aufweist.

12. Zusammensetzung nach einem der Ansprüche 1 bis 11, **dadurch gekennzeichnet, dass** sie 1 bis 10 Gew.-% kolloidale Partikel, bezogen auf das Gesamtgewicht der Zusammensetzung, enthält.

13. Zusammensetzung nach einem der Ansprüche 1, bis 12, **dadurch gekennzeichnet, dass** sie kein Glycerin enthält.

14. Zusammensetzung nach einem der Ansprüche 1 bis 13, **dadurch gekennzeichnet, dass** das nicht flüchtige Öl ausgewählt ist unter: Kohlenwasserstoffölen pflanzlicher Herkunft; Kohlenwasserstoffölen tierischer, mineralischer oder synthetischer Herkunft; Estern von C₈₋₃₀-Fettsäuren und C₃₋₃₀-Monoalkoholen; Hydroxysäuren, deren Säurekette und/oder Alkoholkette 8 bis 29 Kohlenstoffatome aufweist; Pentaerythritestern; geradkettigen oder verzweigten Alkoholen mit 8 bis 26 Kohlenstoffatomen; fluorierten Ölen; Siliconölen; und deren Gemischen.

15. Zusammensetzung nach einem der Ansprüche 1 bis 14, **dadurch gekennzeichnet, dass** das flüchtige Öl ausgewählt ist unter: cyclischen Siliconen, linearen Siliconen mit 2 bis 6 Siliciumatomen, verzweigten Kohlenwasserstoffen, Perfluoralkanen und Perfluormorpholinderivaten.

16. Zusammensetzung nach einem der Ansprüche 1 bis 15, **dadurch gekennzeichnet, dass** sie 1 bis 25 Gew.-% flüchtige und nicht flüchtige Öle insgesamt enthält.

17. Zusammensetzung nach einem der Ansprüche 1 bis 16, **dadurch gekennzeichnet, dass** das Gewichtsverhältnis der flüchtigen und nicht flüchtigen Öle im Bereich von 1;1 bis 3:1 liegt.

18. Zusammensetzung nach einem der Ansprüche 1 bis 17, **dadurch gekennzeichnet, dass** sie als O/W-Emulsion, W/O-Emulsion oder W/O/W-Emulsion vorliegt.

19. Kosmetisches Verfahren zur Pflege oder zum Schminken der Haut des Gesichts und/oder des Halses, insbesondere bei gealterter Haut oder fettiger Haut, das das Auftragen der Zusammensetzung nach einem der Ansprüche 1 bis 18 auf die Haut umfasst.

20. Kosmetische Verwendung der Zusammensetzung nach einem der Ansprüche 1 bis 18, um die Unzulänglichkeiten der Haut, wie Falten, Poren, Unregelmäßigkeiten des Mikroreliefs der Haut und Pigmeritflecken zu verbergen oder zu kaschieren.

21. Kosmetische Verwendung der Zusammensetzung nach einem der Ansprüche 1 bis 18, um die Haut zu mattieren.

22. Kosmetische Verwendung der Zusammensetzung nach einem der Ansprüche 1 bis 18, um die Falten und Fältchen zu glätten und/oder die Haut des Gesichts und/oder des Halses zu straffen.
